(19) Europäisches Patentamt
European Patent Office
Office européen des brevets

(11) Veröffentlichungsnummer: **0 348 746**
**A2**

(12) # EUROPÄISCHE PATENTANMELDUNG

(21) Anmeldenummer: **89110854.0**

(22) Anmeldetag: **15.06.89**

(51) Int. Cl.⁴: **C07D 513/04 , A01N 43/90 , //(C07D513/04,277:00,239:00)**

(30) Priorität: **28.06.88 DE 3821711**

(43) Veröffentlichungstag der Anmeldung:
**03.01.90 Patentblatt 90/01**

(84) Benannte Vertragsstaaten:
**BE CH DE ES FR GB GR IT LI NL**

(71) Anmelder: **BAYER AG**
**D-5090 Leverkusen 1 Bayerwerk(DE)**

(72) Erfinder: **Lindner, Werner, Dr.**
**Märchenstrasse 39**
**D-5000 Köln 80(DE)**
Erfinder: **Brandes, Wilhelm, Dr.**
**Eichendorffstrasse 3**
**D-5653 Leichlingen(DE)**

(54) **Thiazolopyrimidin-Derivate, Verfahren zu ihrer Herstellung und ihre Verwendung als Schädlingsbekämpfungsmittel.**

(57) Die vorliegende Erfindung betrifft neue Thiazolopyrimidin-Derivate der allgemeinen Formel (I)

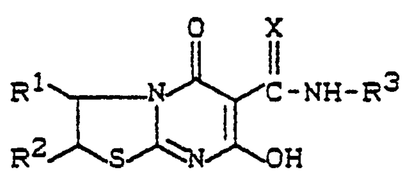

(I)

in welcher
$R^1$ für Wasserstoff oder Alkyl steht,
$R^2$ für Wasserstoff, Alkyl oder gegebenenfalls substituiertes Aryl steht,
$R^3$ für jeweils gegebenenfalls substituiertes Alkyl, Cycloalkyl, Aryl, Arylcarbonyl oder Arylsulfonyl steht, und
X für Sauerstoff oder Schwefel steht,
welche als Schädlingsbekämpfungsmittel verwendet werden können.

EP 0 348 746 A2

# Thiazolopyrimidin-Derivate, Verfahren zu ihrer Herstellung und ihre Verwendung als Schädlingsbekämpfungsmittel

Die vorliegende Erfindung betrifft neue Thiazolopyrimidin-Derivate, ein Verfahren und neue Zwischenprodukte zu ihrer Herstellung sowie ihre Verwendung als Schädlingsbekämpfungsmittel.

Bestimmte Thiazolopyrimidin-Derivate, wie z. B. 2,3,6,7-Tetrahydro-5,7-dioxo-5-thiazolo-[3,2,-a]-pyrimidin, sind bereits aus der Literatur bekannt (vgl. J. Am. Chem. Soc. 64 (1942), 2709 - 2712); jedoch ist über eine Verwendung solcher Verbindungen als Schädlingsbekämpfungsmittel bisher nichts bekannt geworden.

Als Schädlingsbekämpfungsmittel, insbesondere zur Bekämpfung von pilzlichen Pflanzenkrankheiten, ist hingegen z. B. N,N-Dimethyl-N′-phenyl-(N′-fluordichlormethylthio)-sulfamid (Dichlofluanid/Euparen) bekannt (vgl. DE-AS 11 93 498).

Es wurden nun neue Thiazolopyrimidin-Derivate der allgemeinen Formel (I)

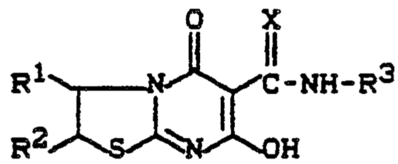

(I)

in welcher

$R^1$ für Wasserstoff oder Alkyl steht,

$R^2$ für Wasserstoff, Alkyl oder gegebenenfalls substituiertes Aryl steht,

$R^3$ für jeweils gegebenenfalls substituiertes Alkyl, Cycloalkyl, Aryl, Arylcarbonyl oder Arylsulfonyl steht und

X für Sauerstoff oder Schwefel steht,

gefunden.

Die Verbindungen der Formel (I) liegen im tautomeren Gleichgewicht vor:

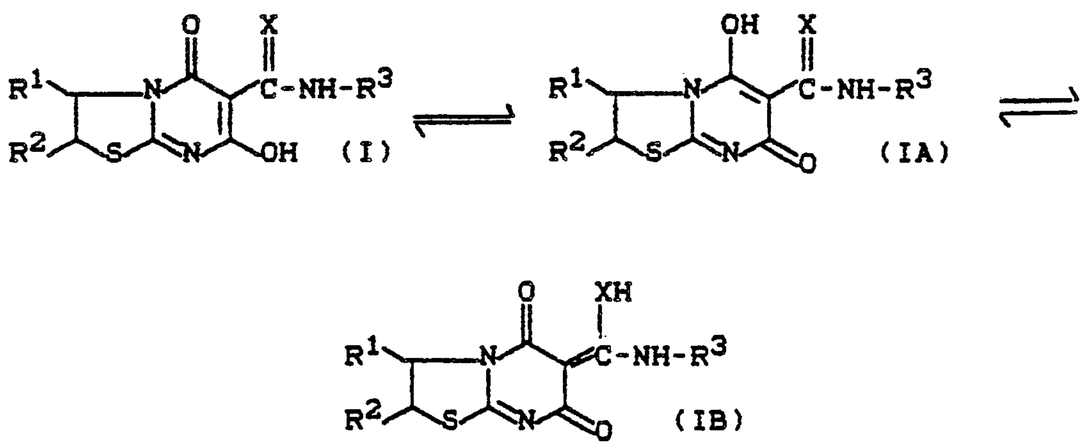

(I) (IA) (IB)

Der Einfachheit halber wird stets von Verbindungen der Formel (I) gesprochen, obwohl die reinen Verbindungen und/oder ihre Gemische mit unterschiedlicher Zusammensetzung gemeint sind und beansprucht werden.

Weiterhin wurde gefunden, daß man die neuen Verbindungen der allgemeinen Formel (I)

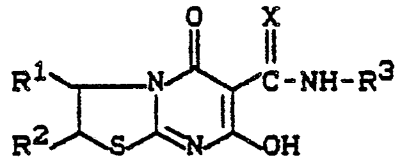

(I)

in welcher

$R^1$ für Wasserstoff oder Alkyl steht,
$R^2$ für Wasserstoff, Alkyl oder gegebenenfalls substituiertes Aryl steht,
$R^3$ für jeweils gegebenenfalls substituiertes Alkyl, Cycloalkyl, Aryl, Arylcarbonyl oder Arylsulfonyl steht und
X für Sauerstoff oder Schwefel steht,
erhält, wenn man Thiazolopyrimidine der allgemeinen Formel (II)

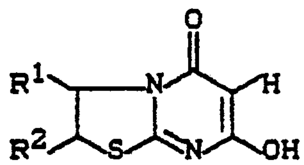

(II)

in welcher
$R^1$ und $R^2$ die oben angegebenen Bedeutungen haben,
mit Iso(thio)cyanaten der allgemeinen Formel (III)
$X = C = N - R^3$ (III)
in welcher
$R^3$ und X die oben angegebenen Bedeutungen haben,
gegebenenfalls in Gegenwart eines Säureakzeptors und gegebenenfalls in Gegenwart eines Verdünnungsmittels umsetzt.

Die neuen Thiazolopyrimidin-Derivate der allgemeinen Formel (I) zeigen starke Wirkung als Schädlingsbekämpfungsmittel, insbesondere als Fungizide, daneben in gewissem Umfang auch als Insektizide.

Überraschenderweise zeigen die neuen Verbindungen der Formel (I) wesentlich stärkere fungizide Wirkung als das bekannte N,N-Dimethyl-N′-phenyl-(N′-fluordichlormethylthio)-sulfamid.

In den allgemeinen Formeln bedeutet Alkyl geradkettiges oder verzweigtes Alkyl mit vorzugsweise 1 bis 6, insbesondere 1 bis 4 und besonders bevorzugt 1 oder 2 Kohlenstoffatomen.

Cycloalkyl der allgemeinen Formeln enthält vorzugsweise 3 bis 7, insbesondere 3, 5 oder 6 Ringglieder. Beispielhaft seien Cyclopropyl, Cyclopentyl und Cyclohexyl genannt.

Aryl der allgemeinen Formeln bedeutet vorzugsweise Phenyl oder Naphthyl, insbesondere Phenyl. Die Arylteile in Arylcarbonyl und Arylsulfonyl sind vorzugsweise Naphthyl und Phenyl, insbesondere Phenyl.

Die gegebenenfalls substituierten Reste der allgemeinen Formeln können durch einen oder mehrere, vorzugsweise 1 bis 3 gleiche oder verschiedene Substituenten substituiert sein.

Als bevorzugte Substituenten seinen genannt:
Halogen, Cyano, Nitro, Alkoxycarbonyl mit 1 bis 4 Kohlenstoffatomen im Alkoxyrest, Phenyl, Phenoxy [welches gegebenenfalls durch eine Trifluormethylgruppe und/oder ein bis 3 Halogenatome substituiert ist], Alkyl mit 1 bis 4 Kohlenstoffatomen, Halogenalkyl mit 1 oder 2 Kohlenstoffatomen und 1 bis 5 gleichen oder verschiedenen Halogenatomen, Alkoxy mit 1 bis 4 Kohlenstoffatomen, Halogenalkoxy mit 1 oder 2 Kohlenstoffatomen und 1 bis 5 gleichen oder verschiedenen Halogenatomen, Alkylthio mit 1 bis 4 Kohlenstoffatomen, Halogenalkylthio mit 1 oder 2 Kohlenstoffatomen und 1 bis 5 gleichen oder verschiedenen Halogenatomen, Alkylsulfinyl mit 1 bis 4 Kohlenstoffatomen, Halogenalkylsulfinyl mit 1 oder 2 Kohlenstoffatomen und 1 bis 5 gleichen oder verschiedenen Halogenatomen, Alkylsulfonyl mit 1 bis 4 Kohlenstoffatomen, Halogenalkylsulfonyl mit 1 oder 2 Kohlenstoffatomen und 1 bis 5 gleichen oder verschiedenen Halogenatomen oder im Falle von Phenyl, eine Benzogruppierung oder eine gegebenenfalls einfach oder zweifach durch Sauerstoff und/oder eine Carbonylgruppe unterbrochene Alkandiylgruppierung mit bis zu 4 Kohlenstoffatomen, welche anelliert ist.

Wo nicht anderes angegeben ist, bedeutet Halogen, Fluor, Chlor, Brom oder Jod, vorzugsweise Fluor, Chlor oder Brom und besonders bevorzugt Fluor oder Chlor.

In den allgemeinen Formeln stehen $R^1$ und $R^2$ vorzugsweise für Wasserstoff.

$R^3$ bedeutet in den allgemeinen Formeln vorzugsweise gegebenenfalls substituiertes Phenyl, welches 1 bis 3 gleiche oder verschiedene der oben angegebenen Substituenten tragen kann. Als besonders bevorzugte Substituenten seien aufgeführt: Halogen (vorzugsweise Chlor), Nitro, $C_1$-$C_4$-Alkyl (vorzugsweise Methyl), $C_1$-$C_4$-Alkoxy (vorzugsweise Methoxy und Ethoxy), $C_1$-$C_4$-Halogenalkyl (vorzugsweise Trifluormethyl), $C_1$-$C_4$-Halogenalkoxy (vorzugsweise Trifluormethoxy), $C_1$-$C_4$-Halogenalkylthio (vorzugsweise Trifluormethylthio und Difluor-chlormethylthio), $C_1$-$C_4$-Halogenalkylsulfonyl (vorzugsweise Trifluormethylsulfonyl) oder Phenoxy, welches seinerseits durch die oben angegebenen Reste substituiert sein kann.

In den allgemeinen Formeln steht X vorzugsweise für Sauerstoff.

Die erfindungsgemäßen Thiazolopyrimidin-Derivate sind durch die Formel (I) allgemein definiert. Bevorzugt sind diejenigen Verbindungen der Formel (I), in welcher

$R^1$ für Wasserstoff oder für geradkettiges oder verzweigtes Alkyl mit 1 bis 6 Kohlenstoffatomen steht,

$R^2$ für Wasserstoff, für geradkettiges oder verzweigtes Alkyl mit 1 bis 6 Kohlenstoffatomen oder für Phenyl steht, welches gegebenenfalls einfach bis dreifach, gleichartig oder verschiedenartig durch Halogen, Cyano, Nitro, Alkyl mit 1 bis 4 Kohlenstoffatomen, Halogenalkyl mit 1 oder 2 Kohlenstoffatomen und 1 bis 5 gleichen oder verschiedenen Halogenatomen, Alkoxy mit 1 bis 4 Kohlenstoffatomen, Halogenalkoxy mit 1 oder 2 Kohlenstoffatomen und 1 bis 5 gleichen oder verschiedenen Halogenatomen, Alkylthio mit 1 bis 4 Kohlenstoffatomen, Halogenalkylthio mit 1 oder 2 Kohlenstoffatomen und 1 bis 5 gleichen oder verschiedenen Halogenatomen, Alkylsulfinyl mit 1 bis 4 Kohlenstoffatomen, Halogen alkylsulfinyl mit 1 oder 2 Kohlenstoffatomen und 1 bis 5 gleichen oder verschiedenen Halogenatomen, Alkylsulfonyl mit 1 bis 4 Kohlenstoffatomen und/oder Halogenalkylsulfonyl mit 1 oder 2 Kohlenstoffatomen und 1 bis 5 gleichen oder verschiedenen Halogenatomen substituiert ist,

$R^3$ für geradkettiges oder verzweigtes Alkyl mit 1 bis 6 Kohlenstoffatomen, welches gegebenenfalls durch Halogen, Cyano oder Alkoxycarbonyl mit 1 bis 4 Kohlenstoffatomen im Alkoxyrest substituiert ist, für Cycloalkyl mit 3 bis 6 Kohlenstoffatomen, welches gegebenenfalls einfach bis dreifach, gleichartig oder verschiedenartig durch Halogen, Cyano und/oder Alkoxycarbonyl mit 1 bis 4 Kohlenstoffatomen im Alkoxyrest substituiert ist, oder für Phenyl, Phenylcarbonyl oder Phenylsulfonyl, welche jeweils gegebenenfalls einfach bis dreifach, gleichartig oder verschiedenartig durch Halogen, Cyano, Nitro, Phenyl, Phenoxy [welches gegebenenfalls durch eine Trifluormethylgruppe und/oder ein bis 3 Halogenatome substituiert ist], Alkyl mit 1 bis 4 Kohlenstoffatomen, Halogenalkyl mit 1 oder 2 Kohlenstoffatomen und 1 bis 5 gleichen oder verschiedenen Halogenatomen, Alkoxy mit 1 bis 4 Kohlenstoffatomen, Halogenalkoxy mit 1 oder 2 Kohlenstoffatomen und 1 bis 5 gleichen oder verschiedenen Halogenatomen, Alkylthio mit 1 bis 4 Kohlenstoffatomen, Halogenalkylthio mit 1 oder 2 Kohlenstoffatomen und 1 bis 5 gleichen oder verschiedenen Halogenatomen, Alkylsulfinyl mit 1 bis 4 Kohlenstoffatomen, Halogenal kylsulfinyl mit 1 oder 2 Kohlenstoffatomen und 1 bis 5 gleichen oder verschiedenen Halogenatomen, Alkylsulfonyl mit 1 bis 4 Kohlenstoffatomen und/oder Halogenalkylsulfonyl mit 1 oder 2 Kohlenstoffatomen und 1 bis 5 gleichen oder verschiedenen Halogenatomen substituiert sind, oder für Phenyl, welches durch eine Benzogruppierung oder durch eine gegebenenfalls einfach oder zweifach durch Sauerstoff und/oder eine Carbonylgruppe unterbrochene Alkandiylgruppierung mit bis zu 4 Kohlenstoffatomen anelliert ist, steht, und

X für Sauerstoff oder Schwefel steht.

Besonders bevorzugt sind diejenigen Verbindungen der Formel (I), in welcher

$R^1$ für Wasserstoff, Methyl, Ethyl, Propyl, Isopropyl, Butyl oder Isobutyl (vorzugsweise für Wasserstoff) steht,

$R^2$ für Wasserstoff, Methyl, Ethyl, Propyl, Isopropyl, Butyl, Isobutyl oder Phenyl steht, welches gegebenenfalls einfach oder zweifach, gleichartig oder verschiedenartig durch Fluor, Chlor, Brom, Cyano, Nitro, Methyl, Ethyl, Trifluormethyl, Methoxy, Ethoxy, Difluormethoxy, Trifluormethoxy, Tetrafluorethoxy, Chlortrifluorethoxy, Methylthio, Ethylthio, Trifluormethylthio, Methylsulfonyl und/oder trifluormethylsulfonyl substituiert ist,

$R^3$ für Cyclohexyl oder für Phenyl oder Phenylsulfonyl, wobei letztere (Phenyl und Phenylsulfonyl) jeweils gegebenenfalls einfach bis dreifach, gleichartig oder verschiedenartig, durch Fluor, Chlor, Brom, Cyano, Nitro, Phenyl, Phenoxy [welches gegebenenfalls durch eine Trifluormethylgruppe und/oder durch 1 bis 3 Fluor- und/oder Chloratome substituiert ist], Methyl, Ethyl, Propyl, Isopropyl, Butyl, Isobutyl, tert-Butyl, Trifluormethyl, Methoxy, Ethoxy, Propoxy, Isopropoxy, Difluormethoxy, Trifluormethoxy, Chlordifluormethoxy, Tetrafluorethoxy, Chlortrifluorethoxy, Methylthio, Ethylthio, Difluormethylthio, Trifluormethylthio, Chlordifluormethylthio, Tetrafluorethylthio, Chlortrifluorethylthio, Methylsulfinyl, Ethylsulfinyl, Trifluormethylsulfinyl, Methylsulfonyl, Ethylsulfonyl und/oder Trifluormethylsulfonyl substituiert sind, oder für Phenyl welches durch eine Benzogruppierung oder durch 1,3-Dioxa-propan-1,3-diyl (Methylendioxy), 1,4-Dioxa-butan-1,4-diyl oder 1,3-Dioxa-butan-1,4-diyl anelliert ist, steht und

X für Sauerstoff steht.

Ganz besonders bevorzugt werden die Verbindungen der Formel (I), in welcher

$R^1$ und $R^2$ für Wasserstoff stehen,

$R^3$ für Phenyl steht, welches durch 1 oder 2, gleiche oder verschiedene Reste der Reihe: Fluor, Chlor, Nitro, Methyl, Trifluormethyl, Methoxy, Trifluormethoxy, Trifluormethylthio oder Difluorchlormethylthio (vorzugsweise Chlor, Methyl und Trifluormethoxy) substituiert sein kann und

X für Sauerstoff steht.

Beispiele für die erfindungsgemäßen Verbindungen der allgemeinen Formel (I) sind in der nachstehenden Tabelle 1 aufgeführt.

**Tabelle 1:** Beispiele für die Verbindungen der Formel (I) (X steht in allen Beispielen für Sauerstoff)

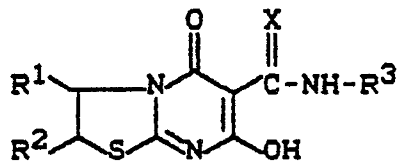

(I)

**Tabelle 1**

| $R^1$ | $R^2$ | $R^3$ |
|---|---|---|
| H | H | 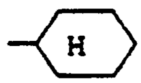 |
| H | H | 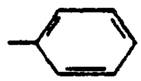 |
| H | H | 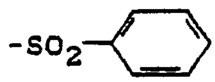 |
| H | H | 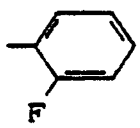 |
| H | H | 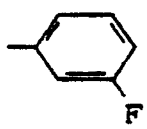 |
| H | H | 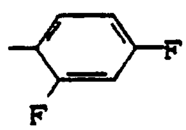 |
| H | H | 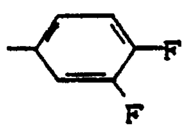 |
| H | H | 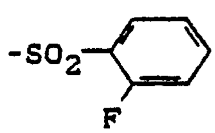 |

Tabelle 1 - Fortsetzung

| $R^1$ | $R^2$ | $R^3$ |
|---|---|---|
| H | H | 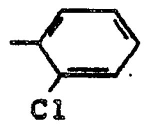 |
| H | H | 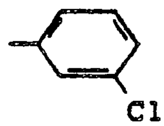 |
| H | H | 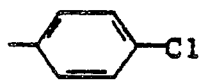 |
| H | H | 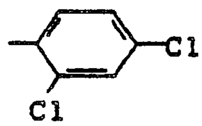 |
| H | H | 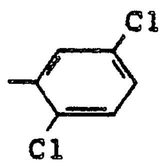 |
| H | H | 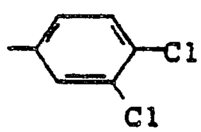 |
| H | H | 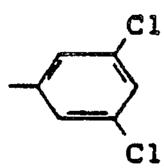 |
| H | H | 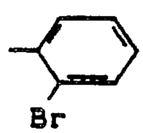 |
| H | H | 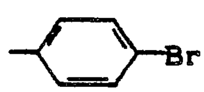 |

__Tabelle 1__ - Fortsetzung

| $R^1$ | $R^2$ | $R^3$ |
|---|---|---|
| H | H | 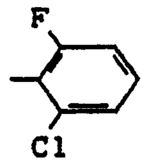 |
| H | H | 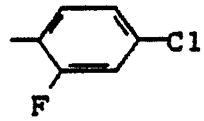 |
| H | H | 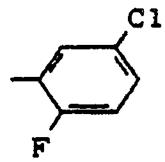 |
| H | H | 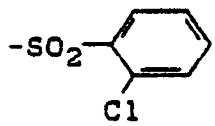 |
| H | H | 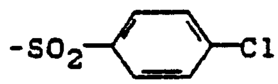 |
| H | H | 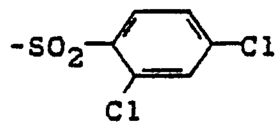 |
| H | H | 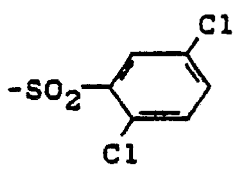 |
| H | H | 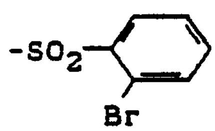 |

Tabelle 1 - Fortsetzung

| $R^1$ | $R^2$ | $R^3$ |
|---|---|---|
| H | H | 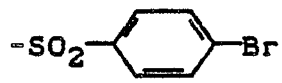 |
| H | H | 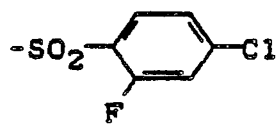 |
| H | H | 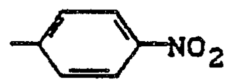 |
| H | H | 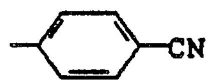 |
| H | H | 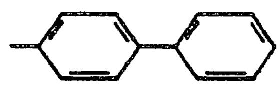 |
| H | H | 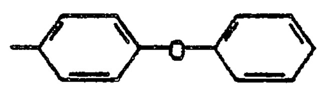 |
| H | H | 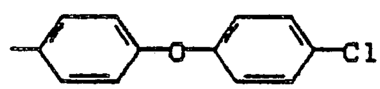 |
| H | H | 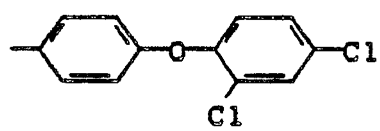 |
| H | H | 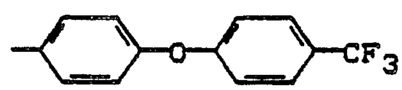 |
| H | H | 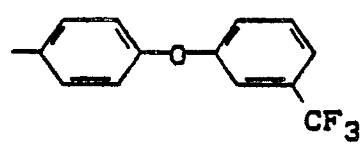 |

Tabelle 1 - Fortsetzung

| $R^1$ | $R^2$ | $R^3$ |
|---|---|---|
| H | H | 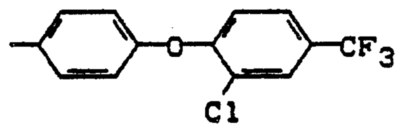 |
| H | H | 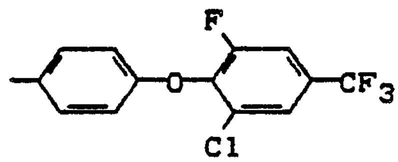 |
| H | H | 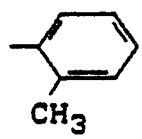 |
| H | H | 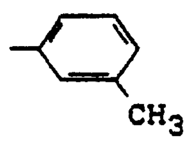 |
| H | H | 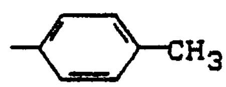 |
| H | H | 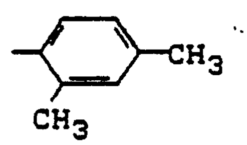 |
| H | H | 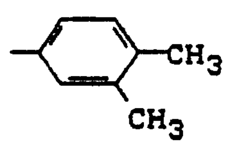 |
| H | H | 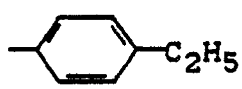 |
| H | H | 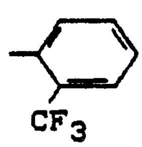 |

<u>Tabelle 1</u> - Fortsetzung

| $R^1$ | $R^2$ | $R^3$ |
|---|---|---|
| H | H | 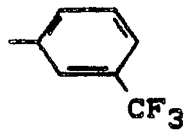 |
| H | H | 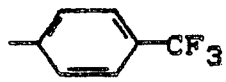 |
| H | H | 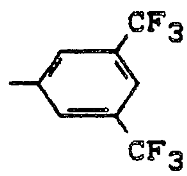 |
| H | H | 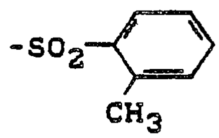 |
| H | H | 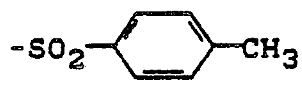 |
| H | H | 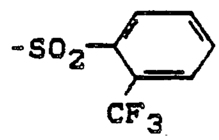 |
| H | H | 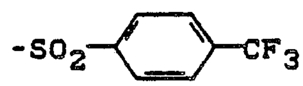 |
| H | H | 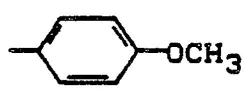 |
| H | H | 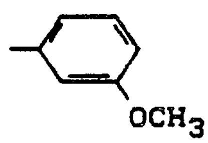 |

**Tabelle 1** - Fortsetzung

| $R^1$ | $R^2$ | $R^3$ |
|---|---|---|
| H | H | 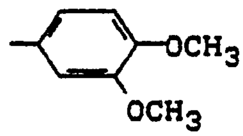 |
| H | H | 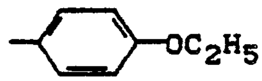 |
| H | H | 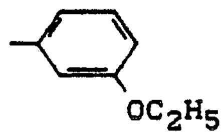 |
| H | H | 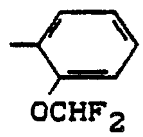 |
| H | H | 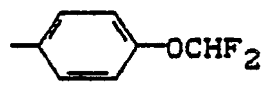 |
| H | H | 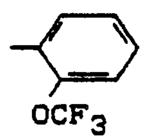 |
| H | H | 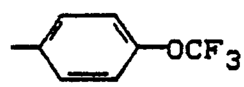 |
| H | H | 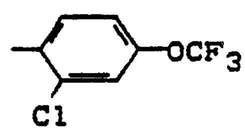 |
| H | H | 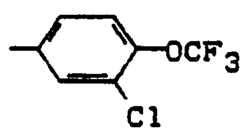 |

<u>Tabelle 1</u> - Fortsetzung

| $R^1$ | $R^2$ | $R^3$ |
|---|---|---|
| H | H | 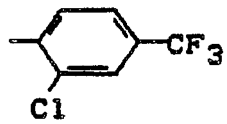 |
| H | H | 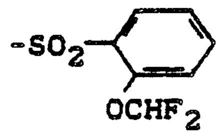 |
| H | H | 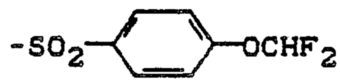 |
| H | H | 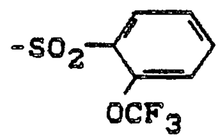 |
| H | H | 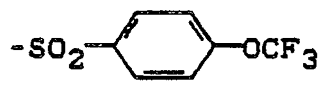 |
| H | H | 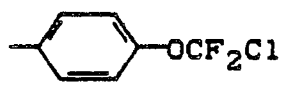 |
| H | H | 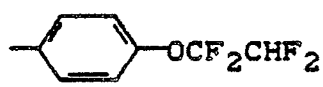 |
| H | H | 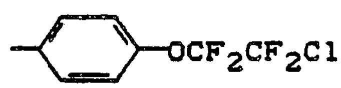 |
| H | H | 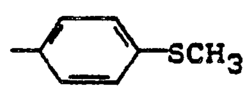 |
| H | H | 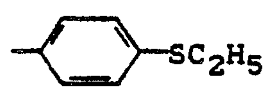 |

Tabelle 1 - Fortsetzung

| $R^1$ | $R^2$ | $R^3$ |
|---|---|---|
| H | H | 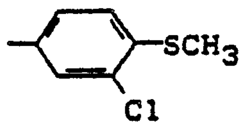 |
| H | H | 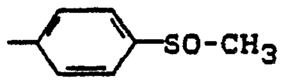 |
| H | H | 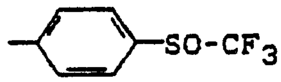 |
| H | H | 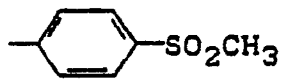 |
| H | H | 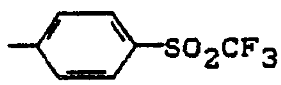 |
| H | H | 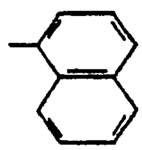 |
| H | H | 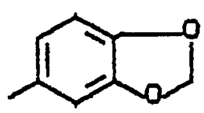 |
| H | H | 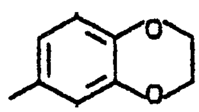 |
| H | H | 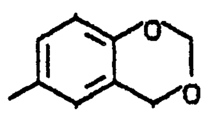 |
| $CH_3$ | 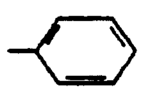 | 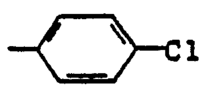 |

Tabelle 1 - Fortsetzung

| $R^1$ | $R^2$ | $R^3$ |
|---|---|---|
| $CH_3$ | 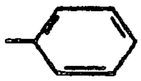 | 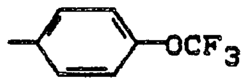 |
| $CH_3$ | 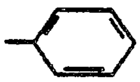 | 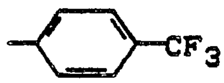 |
| $CH_3$ | 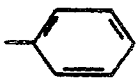 | 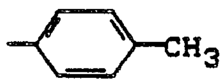 |
| $CH_3$ | 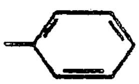 | 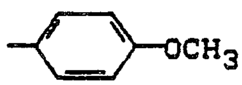 |
| $CH_3$ | 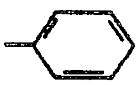 | 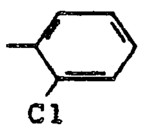 |
| $CH_3$ | 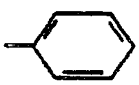 | 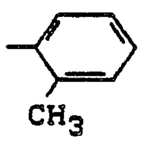 |
| $CH_3$ | 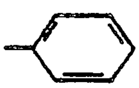 | 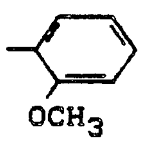 |
| $CH_3$ | 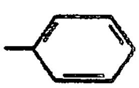 | 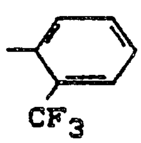 |
| $CH_3$ | 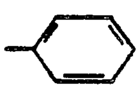 | 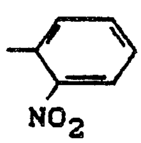 |

<u>Tabelle 1</u> - Fortsetzung

| $R^1$ | $R^2$ | $R^3$ |
|---|---|---|
| $CH_3$ | 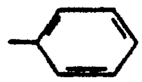 | 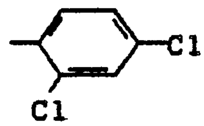 |
| $CH_3$ | 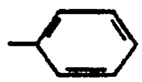 | 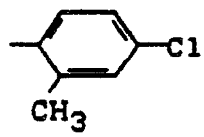 |
| $CH_3$ | 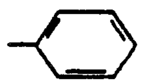 | 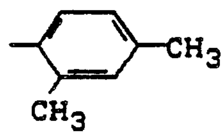 |
| $CH_3$ | 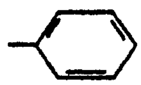 | 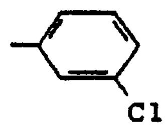 |
| $CH_3$ | 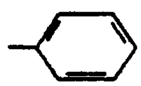 | 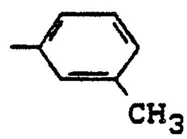 |
| $CH_3$ | 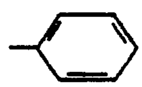 | 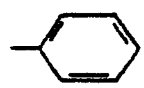 |
| H | 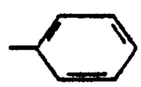 | 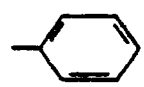 |
| H | 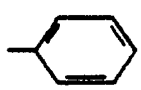 | 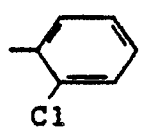 |
| H | 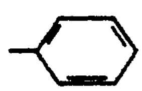 | 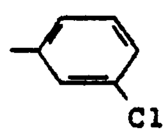 |

Tabelle 1 - Fortsetzung

| $R^1$ | $R^2$ | $R^3$ |
|---|---|---|
| H | 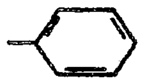 | 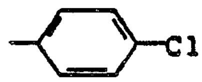 |
| H | 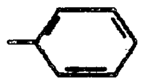 | 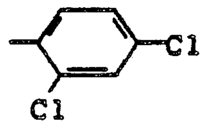 |
| H | 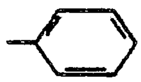 | 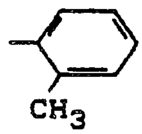 |
| H | 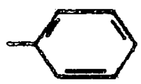 | 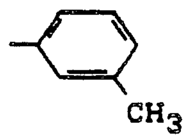 |
| H | 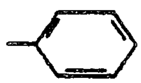 | 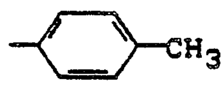 |
| H | 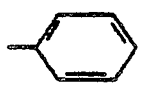 | 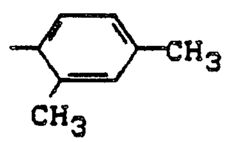 |
| H | 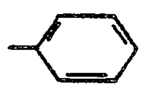 | 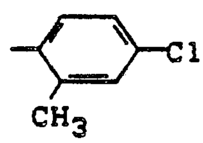 |
| H | 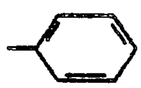 | 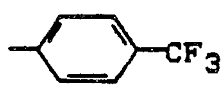 |
| H | 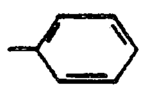 | 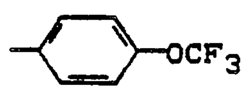 |

**Tabelle 1 - Fortsetzung**

| $R^1$ | $R^2$ | $R^3$ |
|---|---|---|
| H | 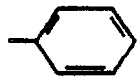 | 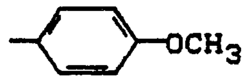 |
| H | 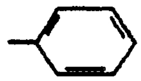 | 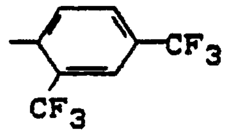 |
| H | 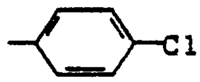 | 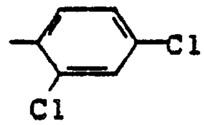 |

Verwendet man beispielsweise 2,3,6,7-Tetrahydro-5,7-dioxo-5-thiazolo-[3,2-a]-pyrimidin und Phenylisocyanat als Ausgangsstoffe, so kann der Reaktionsverlauf beim erfindungsgemäßen Herstellungsverfahren durch das folgende Formelschema wiedergegeben werden:

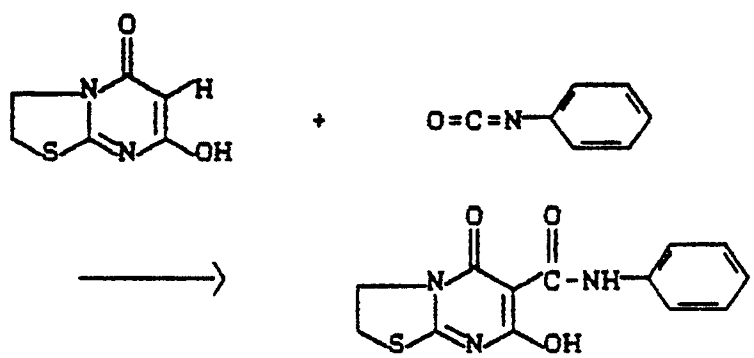

Die beim erfindungsgemäßen Verfahren zur Herstellung von Verbindungen der Formel (I) als Ausgangsstoffe zu verwendenden Thiazolopyrimidine sind durch die Formel (II) allgemein definiert.

In Formel (II) haben $R^1$ und $R^2$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$ und $R^2$ angegeben wurden.

Beispiele für die Ausgangsstoffe der Formel (II) sind in der nachstehenden Tabelle 2 aufgeführt.

**Tabelle 2: Beispiele für die Ausgangsstoffe der Formel (II)**

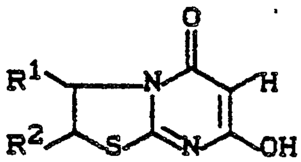

(II)

| $R^1$ | $R^2$ |
|---|---|
| H | H |
| $CH_3$ | $CH_3$ |
| $CH_3$ | H |
| H | $CH_3$ |
| $C_2H_5$ | H |
| $C_3H_7$ | H |
| $C_2H_5$ | $CH_3$ |
| $C_3H_7$ | $CH_3$ |
| $CH_3$ | $C_2H_5$ |
| $CH_3$ | $C_3H_7$ |
| H | $C_2H_5$ |
| H | $C_3H_7$ |
| H | 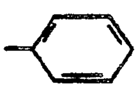 |
| $CH_3$ | 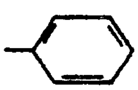 |
| $C_2H_5$ | 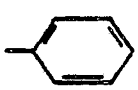 |
| H | 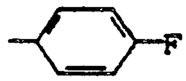 |
| H | 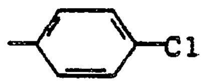 |
| H | 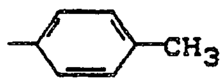 |
| H | 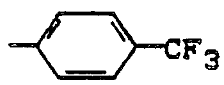 |
| H | 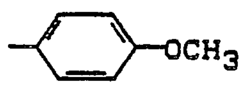 |
| $CH_3$ | 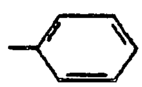 |
| $CH_3$ | 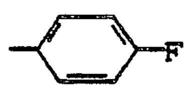 |
| $CH_3$ | 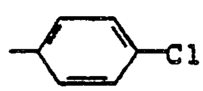 |
| $CH_3$ | 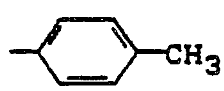 |
| $CH_3$ | 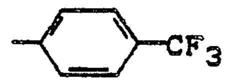 |
| $CH_3$ | 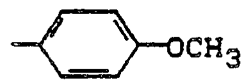 |

Die Ausgangsstofe der Formel (II) sind mit Ausnahme von 2,3,6,7-Tetrahydro-5,7-dioxo-5-thiazolo-[3,2,-a]-pyrimidin (I, $R^1$ = $R^2$ = H) neu und Teil der vorliegenden Erfindung.

Man erhält die neuen Thiazolopyrimidine der Formel (II), wenn man Iminothiazolidine der allgemeinen Formel (IV)

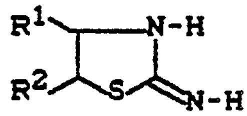

(IV)

in welcher
$R^1$ und $R^2$ die oben angegebenen Bedeutungen haben,
mit Malonsäureestern, wie z. B. Malonsäuredimethylester oder -diethylester, in Gegenwart von Metallen, Metallhydroxiden und/oder Metallalkoholaten, wie z. B. Natrium, Natriumhydroxid, Natrium-methylat und/oder Natriumethylat, in Gegenwart von Verdünnungsmitteln, wie z. B. Methanol und/oder Ethanol, bei Temperaturen zwischen 10 °C und 100 °C umsetzt.

In Formel (IV) haben $R^1$ und $R^2$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$ und $R^2$ angegeben wurden.

Als Beispiele für die Verbindungen der Formel (IV) seien genannt:
2-Imino-4-methyl-thiazolidin, 2-Imino-5-methyl-thiazolidin, 2-Imino-4,5-dimethyl-thiazolidin, 2-Imino-4-ethyl-thiazolidin, 2-Imino-5-ethyl-thiazolidin, 2-Imino-5-phenyl-thiazolidin, 2-Imino-4-methyl-5-phenyl-thiazolidin und 2-Imino-4-ethyl-5-phenyl-thiazolidin.

Die Verbindungen der Formel (IV) sind bekannt und/oder können nach an sich bekannten Verfahren hergestellt werden (vgl. J. Am. Chem. Soc. 80 (1958), 3342; Chem. Abstracts 78 (1973), 43477p; Chem. Abstracts 100 (1984), 85726x).

Die beim erfindungsgemäßen Verfahren weiter als Ausgangsstoffe zu verwendenden Iso(thio)cyanate sind durch die Formel (III) allgemein definiert.

In Formel (III) haben $R^3$ und X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^3$ und X angegeben wurden.

Als Beispiele für die Ausgangsstoffe der Formel (III) seien genannt:
Cyclohexylisocyanat, Phenylisocyanat, Phenylsulfonylisocyanat, 2-Fluor-phenyl- und 2-Fluor-phenylsulfonyl-isocyanat, 3-Fluor-phenyl- und 3-Fluor-phenylsulfonyl-isocyanat, 4-Fluor-phenyl und 4-Fluor-phenylsulfonyl-isocyanat, 2,4-Difluor-phenyl- und 2,4-Difluor-phenylsulfonyl-isocyanat, 3,4-Difluor-phenyl- und 3,4-Difluor-phenylsulfonyl-isocyanat, 2-Chlor-phenyl- und 2-Chlor-phe nylsulfonyl-isocyanat, 3-Chlor- phenyl-und 3-Chlor-phenylsulfonyl-isocyanat, 4-Chlor- phenyl-und 4-Chlor-phenylsulfonyl-isocyanat, 2,4-Dichlor-phenyl- und 2,4-Dichlor-phenylsulfonyl-isocyanat, 2,5-Dichlor-phenyl- und 2,5-Dichlor-phenylsulfonyl-isocyanat, 3,4-Dichlor-phenyl- und 3,4-Dichlor-phenylsulfonyl-isocyanat, 3,5-Dichlor-phenyl- und 3,5-Dichlor-phenylsulfonyl-isocyanat, 2-Brom-phenyl- und 2-Brom-phenylsulfonyl-isocyanat, 3-Brom-phenyl- und 3-Brom-phenylsulfonyl-isocyanat, 4-Brom-phenyl- und 4-Brom-phenylsulfonyl-isocyanat, 2-Methyl-phenyl- und 2-Methyl-phenylsulfonyl-isocyanat, 3-Methyl-phenyl- und 3-Methyl-phenylsulfonyl-isocyanat, 4-Methyl-phenyl- und 4-Methyl-phenylsulfonyl-isocyanat, 2,4-Dimethyl-phenyl-isocyanat, 3,4-Dimethyl-phenyl-isocyanat, 4-Ethyl-phenyl-isocyanat, 2-Trifluormethyl-phenyl- und 2-Trifluormethyl-phenylsulfonyl-isocyanat, 3-Trifluormethyl-phenyl- und 3-Trifluormethyl-phenylsulfonyl-isocyanat, 4-Trifluormethyl-phenyl- und 4-Trifluormethyl-phenylsulfonyl-isocyanat, 2-Methoxy-phenyl- und 2-Methoxy-phenylsulfonyl-isocyanat, 3-Methoxy-phenyl-und 3-Methoxy-phenylsulfonyl-isocyanat, 4-Methoxy-phenyl und 4-Methoxy-phenylsulfonyl-isocyanat, 2-Difluormethoxy-phenyl- und 2-Difluormethoxy-phenylsulfonyl-isocyanat, 4-Difluormethoxy-phenyl- und 4-Difluormethoxy-phenylsulfonyl-isocyanat, 2-Trifluormethoxy-phenyl- und 2-Trifluormethoxy-phenylsulfonyl-isocyanat, 3-Trifluormethoxy-phenyl- und 3-Trifluormethoxy-phenylsulfonyl-isocyanat, 4-Trifluormethoxy-phenyl- und 4-Trifluormethoxy-phenylsulfonyl-isocyanat. 4-Methylthio-phenyl-isocyanat, 4-Ethylthio-phenyl-isocyanat, 1-Naphthyl-isocyanat, 2-Naphthyl-isocyanat und 4-Methylendioxy-phenyl-isocyanat.

Die Ausgangsstoffe der Formel (III) sind bekannt und/oder können nach an sich bekannten Verfahren hergestellt werden (vgl. US-P 4 732 711).

Das erfindungsgemäße Verfahren zur Herstellung der neuen Thiazolopyrimidin-Derivate der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren alle üblicherweise für derartige Umsetzungen verwendbaren Säurebindemittel eingesetzt werden. Vorzugsweise in Frage kommen aliphatische, aromatische oder heterocyclische Amine, beispielsweise Triethylamin, Trimethylamin, Dimethylanilin, Dimethylbenzylamin, Pyridin, 1,5-Diazabicyclo-[4-3,0]-non-5-en (DBN), 1,8-Diazabicyc lo-[5,4,0]-undec-7-en (DBU) und 1,4-Diazabicyclo-[2,2,2]-octan (DABCO).

Die Reaktionstemperaturen können bei dem erfindungsgemäßen Verfahren in einem größeren Bereich variiert werden. Im allgemeinen arbeitet man bei Temperaturen zwischen 0 °C und 100 °C, vorzugsweise bei Temperaturen zwischen 10 °C und 50 °C.

Das erfindungsgemäße Verfahren wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden.

Die erfindungsgemäßen Wirkstoffe weisen eine stark mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis; Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Verbindungen der Formel (I) zeigen insbesondere starke protektive Wirkung gegen Plasmopara-Arten, wie z. B. Plasmopara viticola an Reben, und gegen Botrytis-Arten, wie z. B. Botrytis cinerea an Bohnen. Auch gegen Pyricularia oryzae an Reis ist gute Wirkung zu beobachten.

In gewissem Umfang werden auch tierische Schädlinge, wie z. B. Käfer- und Mückenlarven, bekämpft.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Ge steine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise

von 0,0001 bis 0,02 % am Wirkungsort erforderlich.

Herstellungsbeispiele

Beispiel 1

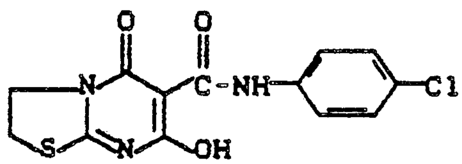

66 ml (0,44 Mol) 1,8-Diazabicyclo-[5,4,0]-undec-7-en (DBU) werden bei 20 °C bis 30 °C unter Rühren zu einer Mischung aus 74 g (0,44 Mol) 2,3,6,7-Tetrahydro-5,7-dioxo-5-thiazolo-[3,2-a]-pyrimidin, 67 g (0,44 Mol) 4-Chlor-phenyl-isocyanat und 1000 ml Tetrahydrofuran tropfenweise gegeben und das Reaktionsgemisch wird noch 60 Minuten gerührt. Anschließend wird langsam mit Wasser auf etwa das doppelte Volumen verdünnt, weitere 15 Minuten gerührt und filtriert. Das Filtrat wird mit konz. Salzsäure angesäuert und das hierbei kristallin angefallene Produkt durch Absaugen isoliert.

Man erhält 130 g (91 % der Theorie) 6-(4-Chlor-phenylamino-carbonyl)-2,3,6,7-tetrahydro-5,7-dioxo-5-thiazolo-[3,2-a]-pyrimidin vom Schmelzpunkt 260 °C.

Analog Beispiel 1 können die in der nachstehenden Tabelle 3 aufgeführten Verbindungen der Formel (I) hergestellt werden.

**Tabelle 3: Herstellungsbeispiele für die Verbindungen der Formel (I)**

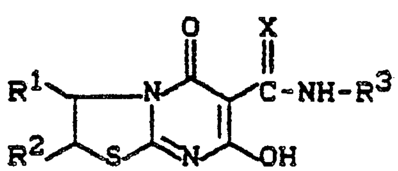

(I)

Tabelle 3

| Bsp.-Nr. | $R^1$ | $R^2$ | $R^3$ | X | Schmelzpunkt (°C) |
|---|---|---|---|---|---|
| 2 | H | H | 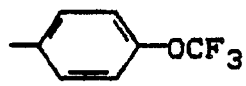 | O | 211 |
| 3 | H | H | 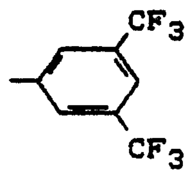 | O | 221 |
| 4 | H | H | 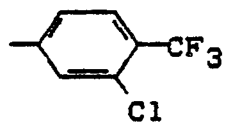 | O | 269 |
| 5 | H | H | 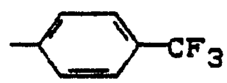 | O | 267 |
| 6 | H | H | 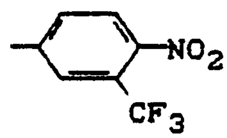 | O | 272 |
| 7 | H | H | 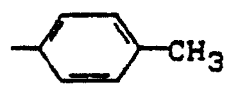 | O | 275 |
| 8 | H | H | 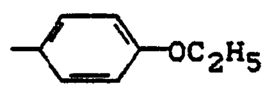 | O | 221 |
| 9 | H | H | 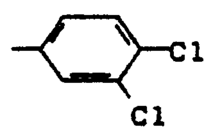 | O | 303 |
| 10 | H | H | 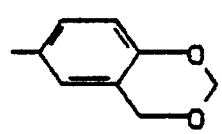 | O | 234 |

<u>Tabelle 3</u> - Fortsetzung

| Bsp.-Nr. | $R^1$ | $R^2$ | $R^3$ | X | Schmelzpunkt (°C) |
|---|---|---|---|---|---|
| 11 | H | H | 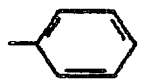 | O | 208 |
| 12 | H | H | 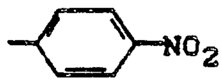 | O | 286 |
| 13 | H | H | 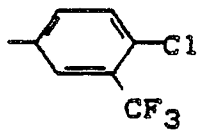 | O | 256 |
| 14 | H | H | 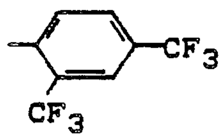 | O | 244 |
| 15 | H | H | 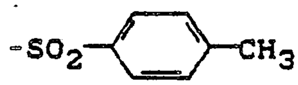 | O | 246 |
| 16 | H | H | 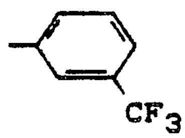 | O | 105 |
| 17 | H | H | 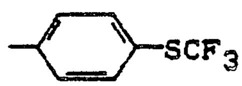 | O | 220 |
| 18 | H | H | 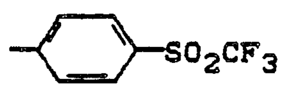 | O | 230 |
| 19 | H | H | 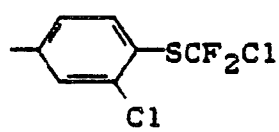 | O | 211 |

Tabelle 3 - Fortsetzung

| Bsp.-Nr. | $R^1$ | $R^2$ | $R^3$ | X | Schmelzpunkt (°C) |
|---|---|---|---|---|---|
| 20 | H | H | 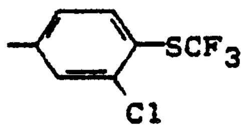 | O | 220 |
| 21 | H | H | 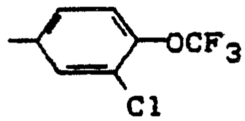 | O | 209 |
| 22 | H | H | 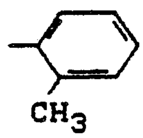 | O | 257 |
| 23 | H | H | 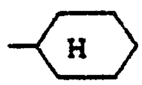 | O | 145 |
| 24 | H | H | 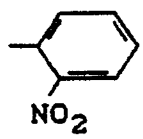 | O | 228 |
| 25 | H | H | 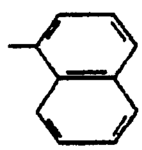 | O | 253 |
| 26 | H | H | 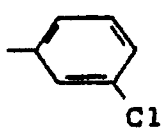 | O | 258 |
| 27 | H | H | 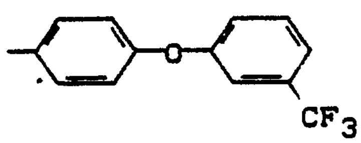 | O | 219 |

<u>Tabelle 3</u> - Fortsetzung

| Bsp.-Nr. | $R^1$ | $R^2$ | $R^3$ | X | Schmelzpunkt (°C) |
|---|---|---|---|---|---|
| 28 | H | H | 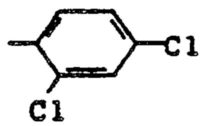 | O | 228 |
| 29 | H | H | 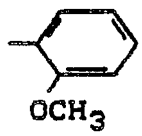 | O | 270 |
| 30 | H | H | 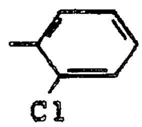 | O | 250 |
| 31 | H | H | 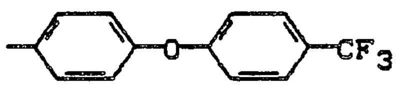 | O | 239 |
| 32 | H | H | 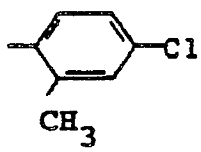 | O | 248 |
| 33 | H | H | 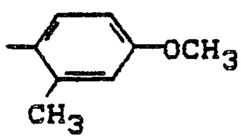 | O | 234 |
| 34 | H | H | 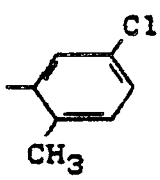 | O | 297 |
| 35 | H | H | 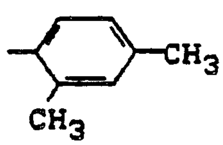 | O | 260 |

<u>Tabelle 3</u> - Fortsetzung

| Bsp.-Nr. | $R^1$ | $R^2$ | $R^3$ | X | Schmelzpunkt (°C) |
|---|---|---|---|---|---|
| 36 | $CH_3$ | $C_6H_5$ | 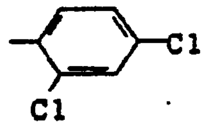 | O | 183 |
| 37 | $CH_3$ | $C_6H_5$ | 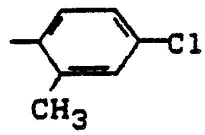 | O | 191 |
| 38 | $CH_3$ | $C_6H_5$ | 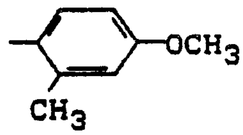 | O | 173 |
| 39 | $CH_3$ | $C_6H_5$ | 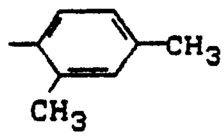 | O | 182 |
| 40 | $CH_3$ | $C_6H_5$ | 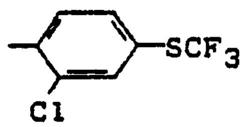 | O | 228 |
| 41 | $CH_3$ | $C_6H_5$ | 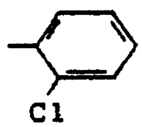 | O | 205 |
| 42 | $CH_3$ | $C_6H_5$ | 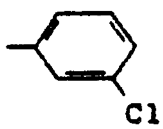 | O | 180 |
| 43 | $CH_3$ | $C_6H_5$ | 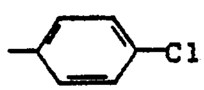 | O | 175 |

**Tabelle 3** - Fortsetzung

| Bsp.-Nr. | $R^1$ | $R^2$ | $R^3$ | X | Schmelzpunkt (°C) |
|---|---|---|---|---|---|
| 44 | $CH_3$ | $C_6H_5$ | 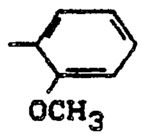 | O | 210 |
| 45 | $CH_3$ | $C_6H_5$ | 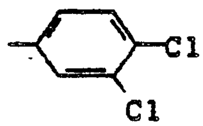 | O | 186 |
| 46 | $CH_3$ | $C_6H_5$ | 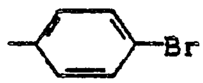 | O | 199 |
| 47 | $CH_3$ | $C_6H_5$ | 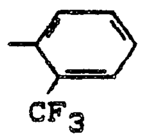 | O | 186 |
| 48 | $CH_3$ | $C_6H_5$ | 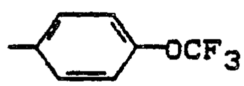 | O | 180 |
| 49 | $CH_3$ | $C_6H_5$ | 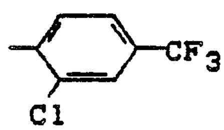 | O | 191 |
| 50 | $CH_3$ | $C_6H_5$ | 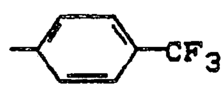 | O | 206 |
| 51 | $CH_3$ | $C_6H_5$ | 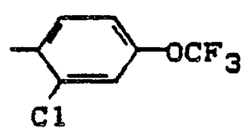 | O | 175 |
| 52 | $CH_3$ | $C_6H_5$ | 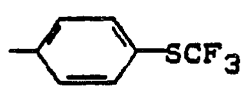 | O | 200 |

**Tabelle 3 - Fortsetzung**

| Bsp.-Nr. | $R^1$ | $R^2$ | $R^3$ | X | Schmelzpunkt (°C) |
|---|---|---|---|---|---|
| 53 | $CH_3$ | $C_6H_5$ | 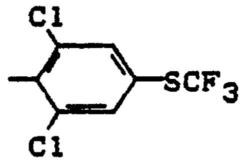 | O | 160 |
| 54 | $CH_3$ | $C_6H_5$ | 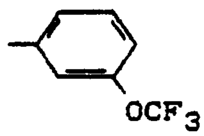 | O | 150 |
| 55 | $CH_3$ | $C_6H_5$ | 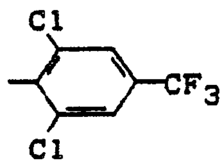 | O | 233 |
| 56 | $CH_3$ | $C_6H_5$ | 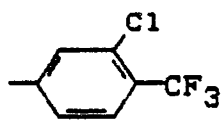 | O | 178 |

Ausgangsstoffe der Formel (II)

Beispiel (II-1)

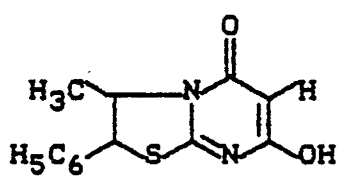

8,4 g (0,37 Mol) Natrium werden zu 200 ml Ethanol gegeben und nach Ende der Umsetzung werden nacheinander 59 g (0,37 Mol) Malonsäurediethylester und eine Lösung von 70 g (0,37 Mol) 2-Imino-4-methyl-5-phenyl-thiazolidin in 400 ml Ethanol dazu gegeben. Das Reaktionsgemisch wird eine Stunde bei 50 °C und weitere 12 Stunden unter Rückfluß gerührt. Nach Abkühlen mit Eis wird mit konz. Salzsäure angesäuert und weiter langsam mit Wasser verdünnt. Das hierbei kristallin angefallene Produkt wird durch Absaugen isoliert.

Man erhält 72 g (76 % der Theorie) 2-Phenyl-3-methyl-2,3,6,7-tetrahydro-5,7-dioxo-5-thiazolo-[3,2-a]-pyrimidin vom Schmelzpunkt 244 °C.

Verwendungsbeispiele

Beispiel A

Plasmopara-Test (Reben) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Plasmopara viticola inokuliert und verbleiben dann 1 Tag in einer Feuchtkammer bei 20 bis 22 °C und 100 % relativer Luftfeuchtigkeit. Anschließend werden die Pflanzen 5 Tage im Gewächshaus bei 22 °C und ca. 80 % Luftfeuchtigkeit aufgestellt. Die Pflanzen werden dann angefeuchtet und 1 Tag in eine Feuchtkammer gestellt.

7 Tage nach der Inokulation erfolgt die Auswertung.

Bei diesem Test zeigte z.B. die Verbindung aus Beispiel 2 bie einer beispielhaften Konzentration von 5 ppm einen Wirkungsgrad von über 90 %.

Beispiel B

Botrytis-Test (Bohne)/protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20 °C aufgestellt. 3 Tage nach der Inokulation wird die Größe der Befallsflecken auf den Blättern ausgewertet.

Bei diesem Test zeigten z.B. die Verbindungen der Beispiele 28 und 32 bei einer beispielhaften Konzentration von 100 ppm einen Wirkungsgrad von über 80 %.

**Ansprüche**

1. Thiazolopyrimidin-Derivate der allgemeinen Formel (I)

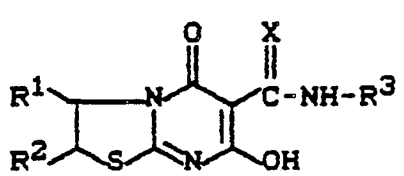

(I)

in welcher
$R^1$ für Wasserstoff oder Alkyl steht,
$R^2$ für Wasserstoff, Alkyl oder gegebenenfalls substituiertes Aryl steht,
$R^3$ für jeweils gegebenenfalls substituiertes Alkyl, Cycloalkyl, Aryl, Arylcarbonyl oder Arylsulfonyl steht, und
X für Sauerstoff oder Schwefel steht, deren tautomeren Formen oder Gemischen unterschiedlicher Zusammensetzung.

2. Thiazolopyrimidin-Derivate der Formel (I) gemäß Anspruch 1,
in welcher
$R^1$ für Wasserstoff oder für geradkettiges oder verzweigtes Alkyl mit 1 bis 6 Kohlenstoffatomen steht,
$R^2$ für Wasserstoff, für geradkettiges oder verzweigtes Alkyl mit 1 bis 6 Kohlenstoffatomen oder für Phenyl

steht, welches gegebenenfalls einfach bis dreifach, gleichartig oder verschiedenartig durch Halogen, Cyano, Nitro, Alkyl mit 1 bis 4 Kohlenstoffatomen, Halogenalkyl mit 1 oder 2 Kohlenstoffatomen und 1 bis 5 gleichen oder verschiedenen Halogenatomen, Alkoxy, mit 1 bis 4 Kohlenstoffatomen, Halogenalkoxy mit 1 oder 2 Kohlenstoffatomen und 1 bis 5 gleichen gleichen oder verschiedenen Halogenatomen, Alkylthio mit 1 bis 4 Kohlenstoffatomen, Halogenalkylthio mit 1 oder 2 Kohlenstoffatomen und 1 bis 5 gleichen oder verschiedenen Halogenatomen, Alkylsulfinyl mit 1 bis 4 Kohlenstoffatomen, Halogenalkylsulfinyl mit 1 oder 2 Kohlenstoffatomen und 1 bis 5 gleichen oder verschiedenen Halogenatomen, Alkylsulfonyl mit 1 bis 4 Kohlenstoffatomen und/oder Halogenalkylsulfonyl mit 1 oder 2 Kohlenstoffatomen und 1 bis 5 gleichen oder verschiedenen Halogenatomen substituiert ist,

$R^{3'}$ für geradkettiges oder verzweigtes Alkyl mit 1 bis 6 Kohlenstoffatomen, welches gegebenenfalls durch Halogen, Cyano oder Alkoxycarbonyl mit 1 bis 4 Kohlenstoffatomen im Alkoxyrest substituiert ist, für Cycloalkyl mit 3 bis 6 Kohlenstoffatomen, welches gegebenenfalls einfach bis dreifach, gleichartig oder verschiedenartig durch Halogen, Cyano und/oder Alkoxycarbonyl mit 1 bis 4 Kohlenstoffatomen im Alkoxyrest substituiert ist, oder für Phenyl, Phenylcarbonyl oder Phenylsulfonyl, welche jeweils gegebenenfalls einfach bis dreifach, gleichartig oder verschiedenartig durch Halogen, Cyano, Nitro, Phenyl, Phenoxy [welches gegebenenfalls durch eine Trifluormethylgruppe und/oder ein bis 3 Halogenatome substituiert ist], Alkyl mit 1 bis 4 Kohlenstoffatomen, Halogenalkyl mit 1 oder 2 Kohlenstoffatomen und 1 bis 5 gleichen oder verschiedenen Halogenatomen, Alkoxy mit 1 bis 4 Kohlenstoffatomen, Halogenalkoxy mit 1 oder 2 Kohlenstoffatomen und 1 bis 5 gleichen oder verschiedenen Halogenatomen, Alkylthio mit 1 bis 4 Kohlenstoffatomen, Halogenalkylthio mit 1 oder 2 Kohlenstoffatomen und 1 bis 5 gleichen oder verschiedenen Halogenatomen, Alkylsulfinyl mit 1 bis 4 Kohlenstoffatomen, Halogenalkylsulfinyl mit 1 oder 2 Kohlenstoffatomen und 1 bis 5 gleichen oder verschiedenen Halogenatomen, Alkylsulfonyl mit 1 bis 4 Kohlenstoffatomen und/ oder Halogenalkylsulfonyl mit 1 oder 2 Kohlenstoffatomen und 1 bis 5 gleichen oder verschiedenen Halogenatomen substituiert sind, oder für Phenyl, welches durch eine Benzogruppierung oder durch eine gegebenenfalls einfach oder zweifach durch Sauerstoff und/oder eine Carbonylgruppe unterbrochene Alkandiylgruppierung mit bis zu 4 Kohlenstoffatomen anelliert ist, steht, und

X für Sauerstoff oder Schwefel steht.

3. Thiazolopyrimidin-Derivate der Formel (I), in welcher

$R^1$ für Wasserstoff, Methyl, Ethyl, Propyl, Isopropyl, Butyl oder Isobutyl steht,

$R^2$ für Wasserstoff, Methyl, Ethyl, Propyl, Isopropyl, Butyl, Isobutyl oder Phenyl steht, welches gegebenenfalls einfach oder zweifach, gleichartig oder verschiedenartig durch Fluor, Chlor, Brom, Cyano, Nitro, Methyl, Ethyl, Trifluormethyl, Methoxy, Ethoxy, Difluormethyoxy, Trifluormethoxy, Tetrafluorethoxy, Chlortrifluorethoxy, Methylthio, Ethylthio, Trifluormethylthio, Methylsulfonyl und/oder Trifluormethylsulfonyl substituiert ist,

$R^3$ für Cyclohexyl oder für Phenyl oder Phenylsulfonyl, wobei letztere (Phenyl und Phenylsulfonyl) jeweils gegebenenfalls einfach bis dreifach, gleichartig oder verschiedenartig, durch Fluor, Chlor, Brom, Cyano, Nitro, Phenyl, Phenoxy [welches gegebenenfalls durch eine Trifluormethylgruppe und/oder durch 1 bis 3 Fluor- und/oder Chloratome substituiert ist], Methyl, Ethyl, Propyl, Isopropyl, Butyl, Isobutyl, tert-Butyl, Trifluormethyl, Methoxy, Ethoxy, Propoxy, Isopropoxy, Difluormethoxy, Trifluormethoxy, Chlordifluormethoxy, Tetrafluorethoxy, Chlortrifluorethoxy, Methylthio, Ethylthio, Difluormethylthio, Trifluormethylthio, Chlordifluormethylthio, Tetrafluorethylthio, Chlortrifluorethylthio, Methylsulfinyl, Ethylsulfinyl, Trifluormethylsulfinyl, Methylsulfonyl, Ethylsulfonyl und/oder Trifluormethylsulfonyl substituiert sind, oder für Phenyl welches durch eine Benzogruppierung oder durch 1,3-Dioxa-propan-1,3-diyl (Methylendioxy), 1,4-Dioxa-butan-1,4,-diyl oder 1,3-Dioxa-butan-1,4-diyl anelliert ist, steht und

X für Sauerstoff steht.

4. Thiazolopyrimidin-Derivate gemäß den Ansprüchen 1 bis 3, in welchen in Formel (I) gemäß Anspruch 1 $R^1$ und $R^2$ für Wasserstoff stehen.

5. Verfahren zur Herstellung der Thiazolopyrimidin-Derivate der allgemeinen Formel (I)

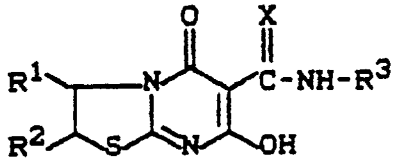

(I)

in welcher

$R^1$ für Wasserstoff oder Alkyl steht,

$R^2$ für Wasserstoff, Alkyl oder gegebenenfalls substituiertes Aryl steht,

$R^3$ für jeweils gegebenenfalls substituiertes Alkyl, Cycloalkyl, Aryl, Arylcarbonyl oder Arylsulfonyl steht, und X für Sauerstoff oder Schwefel steht, deren tautomere Formen oder Gemischen unterschiedlicher Zusammensetzung,
dadurch gekennzeichnet, daß man Thiazolopyrimidine der allgemeinen Formel (II)

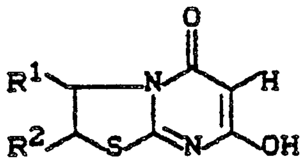

(II)

in welcher
$R^1$ und $R^2$ die oben angegebenen Bedeutungen haben,
mit Iso(thio)cyanaten der allgemeinen Formel (III)
$X = C = N - R^3$ (III)
in welcher
$R^3$ und X die oben angegebenen Bedeutungen haben,
gegebenenfalls in Gegenwart eines Säureakzeptors und gegebenenfalls in Gegenwart eines Verdünnungsmittels umsetzt.

6. Schädlingsbekämpfungsmittel, gekennzeichnet durch einen Gehalt an mindestens einem Thiazolopyrimidin-Derivat der Formel (I) nach den Ansprüchen 1 und 5.

7. Verwendung von substituierten Thiazolopyrimidin-Derivaten der Formel (I) nach den Ansprüchen 1 und 5 zur Bekämpfung von Schädlingen.

8. Verfahren zur Bekämpfung von Schädlingen, dadurch gekennzeichnet, daß man substituierte Thiazolopyrimidin-Derivate der Formel (I) nach den Ansprüchen 1 und 5 auf Schädlinge und/oder ihren Lebensraum einwirken läßt.

9. Verfahren zur Herstellung von Schädlingsbekämpfungsmitteln, dadurch gekennzeichnet, daß man substituierte Thiazolopyrimidin-Derivate der Formel (I) nach den Ansprüchen 1 und 5 mit Streckmitteln und/oder oberflächenaktiven Mitteln vermischt.

10. Thiazolopyrimidine der allgemeinen Formel (II)

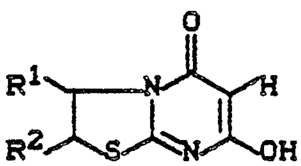

(II)

in welcher
$R^1$ für Wasserstoff oder Alkyl steht,
$R^2$ für Wasserstoff, Alkyl oder gegebenenfalls substituiertes Aryl steht,
wobei wenigstens einer der Reste $R^1$ und $R^2$ von Wasserstoff verschieden ist.